# EUROPEAN PATENT APPLICATION

(11) **EP 4 417 703 A1**
(43) Date of publication of application: **21.08.2024**
(21) Application number: 21960263.8
(22) Date of filing: 15.10.2021
(51) Int. Cl.: C12Q 1/68, C07J 17/00

(54) **USE OF SAPONIN COMPOUND IN NUCLEIC ACID SEQUENCING**

(30) Priority: 11.10.2021 WO PCT/CN2021/123011
(71) Applicant: MGI Tech Co., Ltd., Shenzhen, Guangdong 518083 (CN)
(72) Inventor: JIA, Man, Shenzhen, Guangdong 518083 (CN); MENG, Yixin, Shenzhen, Guangdong 518083 (CN); XU, Chongjun, Shenzhen, Guangdong 518083 (CN); ZHANG, Yinghua, Shenzhen, Guangdong 518083 (CN); WANG, Jingjing, Shenzhen, Guangdong 518083 (CN); GONG, Meihua, Shenzhen, Guangdong 518083 (CN); LI, Jiguang, Shenzhen, Guangdong 518083 (CN)
(74) Representative: Weickmann & Weickmann PartmbB
(86) International application number: PCT/CN2021/123966
(87) International publication number: WO 2023/060527

(57) **Abstract**

The present invention relates to the field of nucleic acid sequencing. In particular, the present invention relates to a scanning reagent containing a saponin compound, a kit containing the scanning reagent and a method for nucleic acid sequencing by means of using the scanning reagent.

## Description

### Technical Field

The present invention relates to the field of nucleic acid sequencing. In particular, the present invention relates to a scanning reagent containing saponin compound, a use of saponin compound as photodamage protectant in nucleic acid sequencing, and a nucleic acid sequencing method.

### Background Art

Sequencing by synthesis is the theoretical basis for sequencing polynucleotides, and it is crucial to identify bases during the sequencing process. Generally, the identification of bases is determined by identifying the type of fluorescent dye labeled on different bases. The process comprises: exposing nucleotides to ultraviolet-visible light to cause the chromophore in fluorescent dye to emit fluorescence, then detecting and photographing fluorescent signals, thereby determining the type of fluorescent dye.

Lasers with wavelengths in the UV-visible range can be used as light sources. However, lasers may cause damage to template nucleotides, which will seriously affect the sequencing quality of polynucleotides.

The buffer solution used in the identification of bases is called as scanning reagent or photography reagent. Laser damage to template nucleotides can be prevented by adding a photodamage protectant to the scanning reagent. However, in the prior art, the concentration of photodamage protectant component in the scanning reagent is relatively high. The use of high-concentration of photodamage protectant may cause the salt precipitation in scanning reagent when it is stored at low temperatures or undergoes long-term sequencing processes. In addition, during long-term storage, some photodamage protectant components may become discolored after being oxidized, thereby affecting the scanning quality and thus the sequencing quality of polynucleotides. In addition, the use of high-concentration of photodamage protectant will increase product costs.

### Contents of the present invention

The present invention uses a saponin compound (e.g., notoginsenoside and ginsenoside) as a photodamage protectant, which can still protect a template nucleotide at a very low working concentration. The saponin compound used in the present invention has no absorption in the ultraviolet and visible light regions, does not interfere with base recognition signals, and will not change color even if it is oxidized during long-term storage.

The present application provides the following inventions:

In one aspect, the present application provides a scanning reagent which comprises a saponin compound and a Tris buffer solution, in which the saponin compound is one or more compounds selected from the group consisting of:

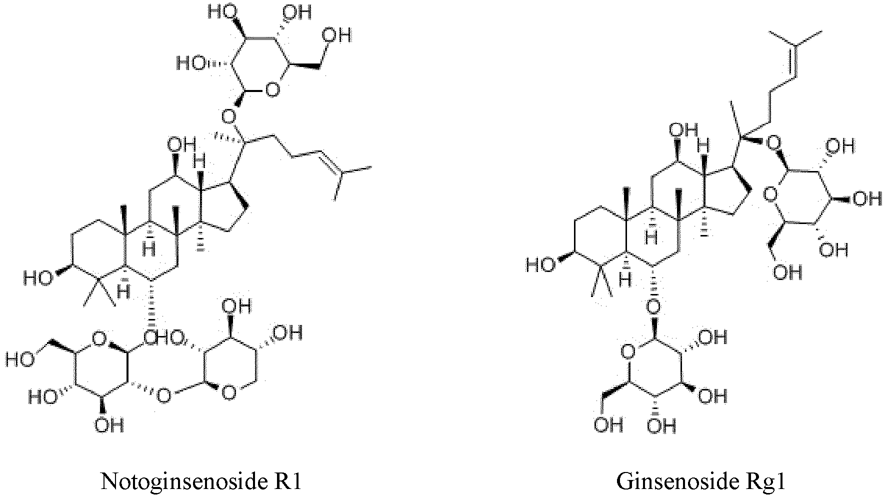

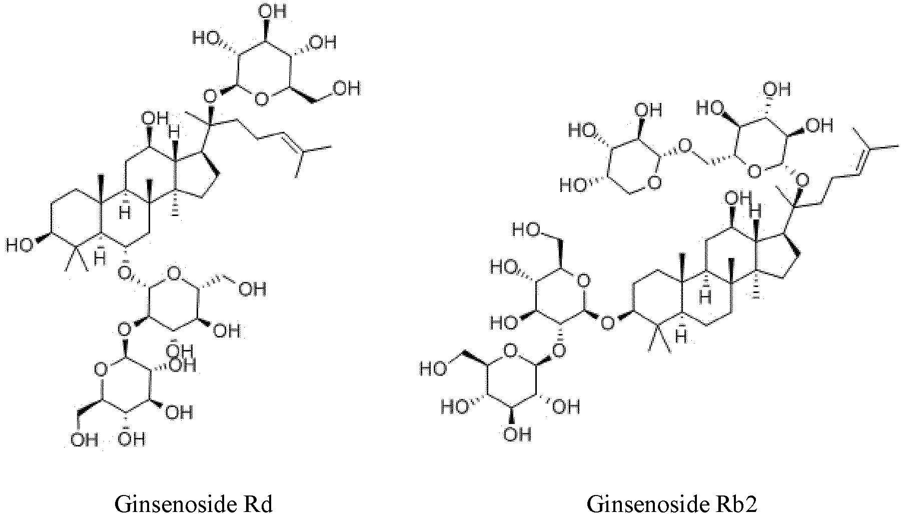

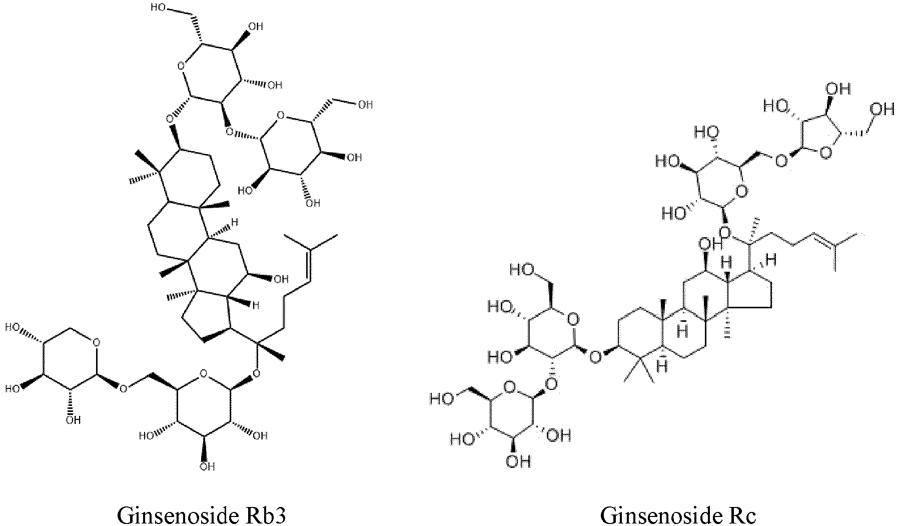

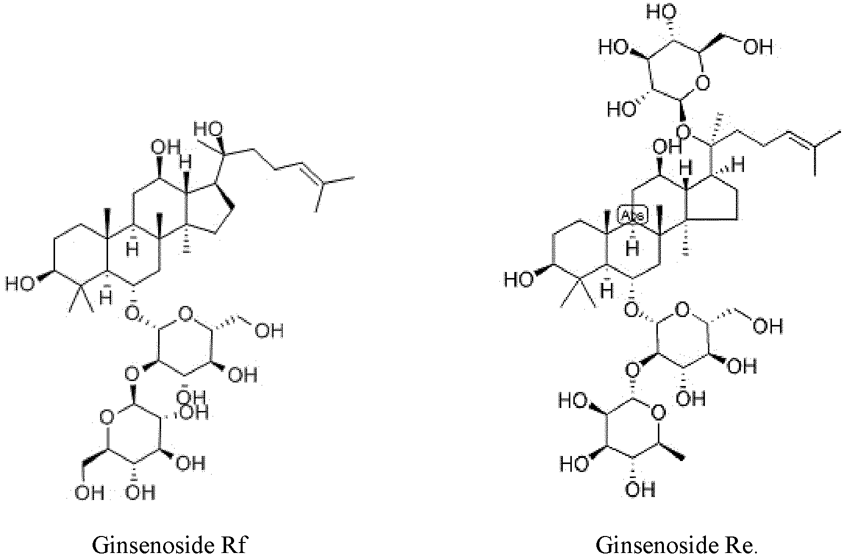

In the present invention, the Tris buffer solution refers to a buffer solution using tris(hydroxymethyl)aminomethane (Tris) as a buffer system.

Tris is widely used in the preparation of buffer solutions used in biochemistry and molecular biology. Tris is a weak base, and the pH of aqueous solution of Tris base is around 10.5. By adding hydrochloric acid to adjust the pH to the desired value, a buffer at that pH can be obtained. Tris and its hydrochloride (Tris-HCl) can also be used to prepare buffer solutions.

The effective buffering range of Tris buffer solutions is between pH 7.0 and 9.2. Tris buffer solution can be used as a solvent to dissolve nucleic acids.

In the present invention, the saponin compound can protect the template nucleotide at a very low concentration. In some embodiments, the saponin compound has a concentration of 0.01-10 mM or 10-100 mM, such as 0.01-0.02 mM, 0.02-0.03 mM, 0.03-0.04 mM, 0.04-0.05 mM, 0.05-0.06 mM, 0.06-0.07 mM, 0.07-0.08 mM, 0.08-0.09 mM, 0.09-0.1 mM, 0.1-0.2 mM, 0.2-0.3 mM, 0.3-0.4 mM, 0.4-0.5 mM, 0.5-0.6 mM, 0.6-0.7 mM, 0.7-0.8 mM, 0.8-0.9 mM, 0.9-1.0 mM, 1-2 mM, 2-3 mM, 3-4 mM, 4-5 mM, 5-6 mM, 6-7 mM, 7-8 mM, 8-9 mM, 9-10 mM, 10-20 mM, 20-30 mM, 30-40 mM, 40-50 mM, 50-60 mM, 60-70 mM, 70-80 mM, 80-90 mM or 90-100 mM.

In some embodiments, the notoginsenoside R1 may have a concentration of 0.01-10 mM, such as 0.01-0.5 mM, 0.5-1 mM, 1-2 mM, 2-3 mM, 3-4 mM, 4-5 mM, 5-6 mM, 6-7 mM, 7-8 mM, 8-9 mM or 9-10 mM.

In some embodiments, the ginsenoside Rg1 may have a concentration of 10-100 mM, such as 10-20 mM, 20-30 mM, 30-40 mM, 40-50 mM, 50-60 mM, 60-70 mM, 70-80 mM, 80-90 mM or 90-100 mM.

In some embodiments, the scanning reagent further comprises an additional photodamage protectant, such as (±)-6-hydroxy-2,5,7,8-tetramethylchromane-2-carboxylic acid (Trolox), ethylenediaminetetraacetic acid tetrasodium salt dihydrate, L-dithiothreitol, or any combination thereof. Adding more than one photodamage protectants to the scanning reagent will be more conducive to improving the quality of DNA sequencing.

In some embodiments, the scanning reagent comprises Trolox, for example, comprises notoginsenoside R1 and Trolox, or comprises ginsenoside Rg1 and Trolox.

In some embodiments, the scanning reagent comprises Trolox and ethylenediaminetetraacetic acid tetrasodium salt dihydrate, for example, comprises notoginsenoside R1, Trolox, and ethylenediaminetetraacetic acid tetrasodium salt dihydrate, or comprises gensenoside Rg1, Trolox and ethylenediaminetetraacetic acid tetrasodium salt dihydrate.

In some embodiments, the scanning reagent comprises Trolox, ethylenediaminetetraacetic acid tetrasodium salt dihydrate, and L-dithiothreitol, for example, comprises notoginsenoside R1, Trolox, ethylenediaminetetraacetic acid tetrasodium salt dihydrate and L-dithiothreitol, or comprises ginsenoside Rg1, Trolox, ethylenediaminetetraacetic acid tetrasodium salt dihydrate and L-dithiothreitol.

In some embodiments, the Trolox, ethylenediaminetetraacetic acid tetrasodium salt dihydrate, or L-dithiothreitol in the scanning reagent each independently has a concentration of 5-50 mM, for example, each independently has a concentration of 5-10 mM, 10-20 mM, 20-30 mM, 30-40 mM, or 40-50 mM.

In some embodiments, the scanning reagent further comprises sodium chloride and/or a DNA stabilizing agent (e.g., Tween-20). The role of sodium chloride is to provide a salt solution background and protect the primers used in sequencing.

In some embodiments, the Tris buffer solution comprises water, tris(hydroxymethyl)aminomethane (Tris Base), sodium chloride, Tween-20, and tris(hydroxymethyl)aminomethane hydrochloride (Tris-HCl).

In some embodiments, the Tris buffer solution is prepared by the following method: dissolving Tris Base (powder), sodium chloride (powder), Tween-20 and Tris-HCl (powder) in certain ratio in ultrapure water.

In some embodiments, in the scanning reagent of the present invention, the concentration of each component is as shown in the table below.

| English name | Concentration range |
|---|---|
| Ethylenediaminetetraacetic Acid Tetrasodium Salt Dihydrate | 5-50 mM |
| (±)-6-Hydroxy-2,5,7, 8-tetramethylchromane-2-carboxylic acid | 5-50 mM |
| Notoginsenoside R1 | 0.01-10 mM |
| L-Dithiothreitol | 5-50 mM |
| Ginsenoside Rg1 | 10-100 mM |

In another aspect, the present application provides a use of a saponin compound (e.g., notoginsenoside or ginsenoside) as a photodamage protectant in nucleic acid sequencing, in which the saponin compound is selected from the group consisting of notoginsenoside R1, ginsenoside Rg1, ginsenoside Rd, ginsenoside Rb2, ginsenoside Rb3, ginsenoside Rc, ginsenoside Rf, and ginsenoside Re, and the structural formulas of each compound are as shown above.

In another aspect, the present application provides a kit, which comprises the scanning reagent of the present invention.

In some embodiments, the kit of the present invention may further comprise: a reagent for immobilizing a nucleic acid molecule to be sequenced to a support (e.g., immobilizing by a covalent or non-covalent linkage); a primer for initiating nucleotide polymerization reaction; a polymerase for nucleotide polymerization reaction; one or more buffer solutions; one or more washing solutions; or any combination thereof.

In some embodiments, the kit of the present invention may further comprise a reagent and/or device for extracting a nucleic acid molecule from a sample. Methods for extracting nucleic acid molecules from samples are well known in the art. Therefore, various reagents and/or devices for extracting nucleic acid molecules can be configured in the kit of the present invention as needed, examples may include reagents for disrupting cells, reagents for precipitating DNA, reagents for washing DNA, reagents for dissolving DNA, reagents for precipitating RNA, reagents for washing RNA, reagents for dissolving RNA, reagents for removing proteins, reagents for removing DNA (e.g., when the target nucleic acid molecule is RNA), reagents for removing RNA (e.g., when the target nucleic acid molecule is DNA), and any combination thereof.

In some embodiments, the kit of the present invention further comprises a reagent for pretreating nucleic acid molecules. In the kit of the present invention, the reagent for pretreating nucleic acid molecules is not subject to additional restrictions and can be selected according to actual needs. The reagent for pretreating nucleic acid molecules comprises, for example, a reagent for fragmenting a nucleic acid molecule (e.g., DNase I), a reagent for complementing a nucleic acid molecule's end (e.g., DNA polymerase, such as T4 DNA polymerase, Pfu DNA polymerase, Klenow DNA polymerase), an adapter molecule, a label molecule, a reagent for linking an adapter molecule to a nucleic acid molecule of interest (e.g., ligase, such as T4 DNA ligase), a reagent for repairing a nucleic acid's end (e.g., DNA polymerase that loses 3'-5' exonuclease activity but displays 5'-3' exonuclease activity), a reagent for amplifying a nucleic acid molecule (e.g., DNA polymerase, primer, dNTP), a reagent for separating and purifying nucleic acid molecules (e.g., chromatography columns), and any combination thereof.

In some embodiments, the kit of the present invention further comprises a support for immobilizing the nucleic acid molecule to be sequenced. Under normal circumstances, the support used to immobilize the nucleic acid molecules to be sequenced is in a solid phase to facilitate handling. Therefore, in this disclosure, a "support" is sometimes also referred to as a "solid support" or "solid phase support." However, it should be understood that the "support" mentioned herein is not limited to a solid, but may also be a semi-solid (e.g., gel).

As used herein, the terms "loaded," "immobilized," and "attached" when used with reference to a nucleic acid, mean direct or indirect attachment to a solid support via a covalent or non-covalent bond. In some embodiments of the present disclosure, the method of the present invention comprises immobilizing a nucleic acid on a solid support via covalent attachment. Typically, however, all that is required is that the nucleic acid remains immobilized or attached to the solid support under the conditions in which using the solid support is desired (e.g., in an application requiring nucleic acid amplification and/or sequencing). In some embodiments, immobilizing the nucleic acid on the solid support may comprise immobilizing an oligonucleotide to be used as a capture primer or amplification primer on the solid support such that the 3' end is available for enzymatic extension, and at least part of the primer sequence is capable of hybridizing to a complementary nucleic acid sequence; the nucleic acid to be immobilized is then hybridized to the oligonucleotide, in which case the immobilized oligonucleotide or polynucleotide can be in 3'- 5' direction. In some embodiments, immobilizing a nucleic acid on a solid support may comprise binding a nucleic acid binding protein to the solid support via amination modification, and capturing the nucleic acid molecule via the nucleic acid binding protein. Alternatively, loading may occur by means other than base pairing hybridization, such as covalent attachment as described above. Non-limiting examples of means of attachment of nucleic acids to solid supports comprise nucleic acid hybridization, biotin-streptavidin binding, sulfhydryl binding, photoactivated binding, covalent binding, antibody-antigen, physical confinement via hydrogel or other porous polymers, etc. Various exemplary methods for immobilizing nucleic acids on solid supports can be found, for example, in G. Steinberg-Tatman et al., Bioconjugate Chemistry 2006, 17, 841-848; Xu X. et al. Journal of the American Chemical Society 128 (2006) 9286-9287; US patent applications US 5639603, US 5641658, US2010248991; international patent applications WO 2001062982, WO 2001012862, WO 200711937, WO0006770; for all intents and purposes, especially for all teachings to the preparation and formation of solid supports on which nucleic acids are fixed, the above documents are incorporated herein by reference in their entirety.

In the present invention, the support may be made of various suitable materials. Such materials include, for example, inorganics, natural polymers, synthetic polymers, and any combination thereof. Specific examples include, but are not limited to: cellulose, cellulose derivatives (e.g., nitrocellulose), acrylics, glass, silica gel, silica, polystyrene, gelatin, polyvinylpyrrolidone, copolymers of vinyl and acrylamide, polystyrene cross-linked with divinylbenzene, etc. (see, for example, Merrifield Biochemistry 1964, 3, 1385-1390), polyacrylamide, latex, dextran, rubber, silicon, plastics, natural sponges, metals plastics, cross-linked dextran (e.g., Sephadex^{™}), agarose gel (e.g., Sepharose^{™}), and other supports known to those skilled in the art.

In some preferred embodiments, the support used to immobilize nucleic acid molecules to be sequenced may be a solid support comprising an inert substrate or matrix (e.g., slide, polymer beads, etc.), the inert substrate or matrix has been functionalized, for example, by the application of an intermediate material containing an active group that allows the covalent linkage with a biomolecule such as polynucleotide. Examples of such supports include, but are not limited to, polyacrylamide hydrogel supported on an inert substrate such as glass, in particular those described in WO 2005/065814 and US 2008/0280773, wherein the contents of the aforementioned patent applications are incorporated herein by reference in their entirety. In such embodiments, the biomolecule (e.g., polynucleotide) can be directly covalently linked to an intermediate material (e.g., hydrogel), which itself can be non-covalently linked to a substrate or matrix (e.g. glass substrate). In some preferred embodiments, the support is a glass slide or silicon chip whose surface is modified with a layer of avidin, amino, acrylamide silane or aldehyde chemical groups.

In the present invention, the support or solid support is not limited to its size, shape and configuration. In some embodiments, the support or solid support is a planar structure, such as a slide, chip, microchip, and/or array. The surface of such support may be in the form of a planar layer.

In some preferred embodiments, the support used to immobilize the nucleic acid molecule to be sequenced is an array of beads or wells (which is also referred to as a chip). The array may be prepared using any of the materials outlined herein for preparing solid supports, and preferably the surface of the beads or wells on the array is functionalized to facilitate the immobilization of nucleic acid molecules. The number of beads or wells on the array is not limited. For example, each array may comprise 10 to 10², 10² to 10³, 10³ to 10⁴, 10⁴ to 10⁵, 10⁵ to 10⁶ , 10⁶ to 10⁷, 10⁷ to 10⁸, 10⁸ to 10⁹ , 10¹⁰ to 10¹¹, 10¹¹ to 10¹², or more beads or wells. In some exemplary embodiments, one or more nucleic acid molecules can be immobilized on the surface of each bead or well. Accordingly, each array can immobilize 10 to 10², 10² to 10³, 10³ to 10⁴, 10⁴ to 10⁵, 10⁵ to 10⁶ , 10⁶ to 10⁷, 10⁷ to 10⁸, 10⁸ to 10⁹ , 10¹⁰ to 10¹¹, 10¹¹ to 10¹², or more nucleic acid molecules. Therefore, such array may be particularly advantageously used for high-throughput sequencing of nucleic acid molecules.

In some preferred embodiments, the kit of the present invention further comprises a reagent for immobilizing a nucleic acid molecule to be sequenced to a support (e.g., immobilization via a covalent or non-covalent linkage). Such reagent comprises, for example, a reagent for activating or modifying the nucleic acid molecule (e.g., its 5' end), such as phosphoric acid, thiol, amine, carboxylic acid, or aldehyde; a reagent for activating or modifying the surface of the support, such as amino-alkoxysilane (e.g., aminopropyltrimethoxysilane, aminopropyltriethoxysilane, 4-aminobutyltriethoxysilane, etc.); a cross-linking agent, such as succinic anhydride, phenyl diisothiocyanate (Guo et al., 1994), maleic anhydride (Yang et al., 1998), 1-ethyl-3-(3-dimethylaminopropyl)-carbodiimide hydrochloride (EDC), m-maleimidobenzoyloxy-N-hydroxysuccinimide (MBS), N-succinimidyl [4-iodoacetyl]aminobenzate (SIAB), succinimidyl 4-(N-maleimidomethyl)cyclohexane-1-carboxylate (SMCC), N-γ-maleimidobutyryloxy-succinimide (GMBS), succinimidyl 4-(p-maleimidophenyl)butyrate (SMPB); and any combination thereof.

In some preferred embodiments, the kit of the present invention further comprises a primer for initiating nucleotide polymerization reaction. In the present invention, the primer is not subject to additional restrictions as long as it can specifically anneal to a region of the target nucleic acid molecule. In some exemplary embodiments, the primer may have a length of 5-50 bp, such as 5-10, 10-15, 15-20, 20-25, 25-30, 30-35, 35-40, 40-45, 45-50 bp. In some exemplary embodiments, the primer may comprise naturally occurring or non-naturally occurring nucleotides. In some exemplary embodiments, the primer comprises or consists of naturally occurring nucleotides. In some exemplary embodiments, the primer comprises modified nucleotides, such as locked nucleic acids (LNA). In some preferred embodiments, the primer comprises universal primer sequences.

In some preferred embodiments, the kit of the present invention further comprises a polymerase for performing nucleotide polymerization reaction. In the present invention, various suitable polymerases may be used to carry out the polymerization reaction. In some exemplary embodiments, the polymerase is capable of synthesizing a new DNA strand using DNA as a template (e.g., DNA polymerase). In some exemplary embodiments, the polymerase is capable of synthesizing a new DNA strand using RNA as a template (e.g., reverse transcriptase). In some exemplary embodiments, the polymerase is capable of synthesizing a new RNA strand using DNA or RNA as a template (e.g., RNA polymerase). Thus, in some preferred embodiments, the polymerase is selected from the group consisting of DNA polymerase, RNA polymerase, and reverse transcriptase.

In some preferred embodiments, the kit of the present invention further comprises one or more excision reagents. In some embodiments, the excision reagent is selected from the group consisting of endonuclease IV and alkaline phosphatase.

In some preferred embodiments, the kit of the present invention further comprises one or more buffer solutions. Such buffer solutions comprise, but are not limited to, buffer solutions for DNase I, buffer solutions for DNA polymerase, buffer solutions for ligase, buffer solutions for elution of nucleic acid molecules, and buffer solutions for dissolving nucleic acid molecules, buffer solutions for nucleotide polymerization reactions (e.g., PCR), and buffer solutions for ligation reactions. The kit of the present invention may comprise any one or more of the above buffer solutions.

In some embodiments, the buffer solution for DNA polymerase comprises a monovalent salt ion (e.g., sodium ion, chloride ion) and/or a divalent salt ion (e.g., magnesium ion, sulfate ion, manganese ion). In some embodiments, the monovalent salt ion or divalent salt ion in the buffer solution has a concentration of 10 µM to 200 mM, such as 10 µM, 50 µM, 100 µM, 200 µM, 500 µM, 1 mM, 3 mM, 10 mM, 20 mM, 50 mM, 100 mM, 150 mM or 200 mM.

In some embodiments, the buffer solution for DNA polymerase comprises trishydroxymethylaminomethane (Tris). In some embodiments, the Tris in the buffer solution has a concentration of 10 mM to 200 mM, such as 10 mM, 20 mM, 50 mM, 100 mM, 150 mM or 200 mM.

In some embodiments, the buffer solution for DNA polymerase comprises an organic solvent, such as DMSO or glycerol (glycerol). In some embodiments, the organic solvent in the buffer solution has a mass content of 0.01% to 10%, such as 0.01%, 0.02%, 0.05%, 1%, 2%, 5% or 10%.

In some embodiments, the buffer solution for DNA polymerase has a pH of 7.0 to 9.0, such as 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9 or 9.0.

In some embodiments, the buffer solution for DNA polymerase comprises: a monovalent salt ion (e.g., sodium ion, chloride ion), a divalent salt ion (e.g., magnesium ion, sulfate ion, manganese ion), Tris and an organic solvent (e.g., DMSO or glycerol). In some embodiments, the buffer solution phase has a pH of 8.8.

In some preferred embodiments, the kit of the present invention further comprises one or more washing solutions. Examples of such wash solutions comprise, but are not limited to, phosphate buffer, citrate buffer, Tris-HCl buffer, acetate buffer, carbonate buffer, and the like. The kit of the present invention may comprise any one or more of the above-mentioned washing solutions.

In another aspect, the present application provides a use of the scanning reagent or kit according to the present invention for sequencing a target polynucleotide.

In another aspect, the present application provides a method for preparing the scanning reagent of the present invention, which comprises dissolving each of the components of the scanning reagent in ultrapure water to form a transparent and uniform solution, and then filtering the solution to obtain the scanning reagent of the present invention. Ultrasound can be used to assist dissolution.

In another aspect, the present application provides a method for sequencing a nucleic acid, which comprises using the scanning reagent of the present invention. In some embodiments, the sequencing method of the present invention comprises synthesizing a growing polynucleotide complementary to a target single-stranded polynucleotide while performing scanning, photographing and detection.

In some embodiments, the method of determining a sequence of a single-stranded polynucleotide of interest comprises:
(a) providing a duplex, a nucleotide, a polymerase, and an excision reagent; the duplex comprises a growing nucleic acid chain and a nucleic acid molecule to be sequenced;
(b) performing a reaction cycle comprising the following Steps (i), (ii) and (iii):
   Step (i): using the polymerase to incorporate nucleotides into the growing nucleic acid chain to form a nucleic acid intermediate containing a blocking group and a detectable label;
   Step (ii): detecting the detectable label on the nucleic acid intermediate;
   Step (iii): using the excision reagent to remove the blocking group on the nucleic acid intermediate.

In some embodiments, the reaction cycle further comprises Step (iv): using an excision reagent to remove the detectable label on the nucleic acid intermediate.

In some embodiments, the method comprises the following steps:
Step 1, loading a DNA nanoball (DNB) onto a prepared sequencing chip;
Step 2, pumping a prepared mixed solution of dNTP molecules into the chip, and using a DNA polymerase to incorporate dNTPs to a complementary strand of a DNA to be tested;
Step 3, taking a photo and scanning, in which since dNTPs are modified molecules with fluorescent groups, lasers with excitation wavelength are used for taking photos; and since lasers have a photodamage effect on DNA, a scanning reagent containing a photodamage protectant is added during the step of taking photo;
Step 4, using an excision reagent to remove the base-terminal fluorescent groups and the 3'-blocking group, and eluting to leave the 3'-OH exposed for the next round of reaction;
Step 5, analyzing the photo results to determine the base sequence of the nucleic acid molecule to be tested.

In the present invention, nucleic acids may comprise nucleotides or nucleotide analogs. Nucleotide usually comprises a saccharide, a nucleobase, and at least one phosphate group. Nucleotides include deoxyribonucleotides, modified deoxyribonucleotides, ribonucleotides, modified ribonucleotides, peptide nucleotides, modified peptide nucleotides, modified phosphate saccharide backbone nucleotides and mixtures thereof. Examples of nucleotides include, for example, adenosine monophosphate (AMP), adenosine diphosphate (ADP), adenosine triphosphate (ATP), thymidine monophosphate (TMP), thymidine diphosphate (TDP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine triphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP) and deoxyuridine triphosphate (dUTP). Nucleotide analogs containing modified nucleobases may also be used in the methods described herein. Exemplary modified nucleobases that may be comprised in polynucleotides, whether with native backbones or similar structures, include, for example, inosine, xanthine, hypoxanthine, isocytosine, isoguanine, 2-aminopurine, 5-methylcytosine, 5-hydroxymethylcytosine, 2-aminoadenine, 6-methyladenine, 6-methylguanine, 2-propylguanine, 2-propyladenine, 2-thiouracil, 2-thiothymine, 2-thiocytosine, 15-halogenated uracil, 15-halogenated cytosine, 5-propynyluracil, 5-propynylcytosine, 6-azouracil, 6-azocytosine, 6-azothymine, 5-uracil, 4-thiouracil, 8-halogenated adenine or guanine, 8-aminoadenine or guanine, 8-thioadenine or guanine, 8-thioalkyladenine or guanine, 8-hydroxyadenine or guanine, 5-halogenated uracil or cytosine, 7-methylguanine, 7-methyladenine, 8-azaguanine, 8-azaadenine, 7-deazaguanine, 7-deazaadenine, 3-deazaguanine, 3-deazaadenine, etc. As known in the art, certain nucleotide analogs cannot be incorporated into polynucleotides, for example, nucleotide analogs such as adenosine 5'-phosphoryl sulfate.

In the method of the present invention, the nucleic acid molecules to be sequenced are not limited by their length. In some preferred embodiments, the length of the nucleic acid molecule to be sequenced can be at least 10 bp, at least 20 bp, at least 30 bp, at least 40 bp, at least 50 bp, at least 100 bp, at least 200 bp, at least 300 bp, at least 400 bp, at least 500 bp, at least 1000 bp, or at least 2000 bp. In some preferred embodiments, the length of the nucleic acid molecule to be sequenced can be 10-20 bp, 20-30 bp, 30-40 bp, 40-50 bp, 50-100 bp, 100-200 bp, 200-300 bp, 300-400 bp, 400-500 bp, 500-1000 bp, 1000-2000 bp, or more than 2000 bp. In some preferred embodiments, the nucleic acid molecule to be sequenced may have a length of 10-1000 bp to facilitate high-throughput sequencing.

In some preferred embodiments, the nucleic acid molecule may be pretreated prior to immobilizing the nucleic acid molecule on the support. Such pretreating comprises, but is not limited to, fragmentation of nucleic acid molecule, end filling-in, addition of adapters, addition of tags, amplification of nucleic acid molecules, isolation and purification of nucleic acid molecules, and any combination thereof.

The term "nanoball" as used herein generally refers to a macromolecule or complex having a compact (approximately) spherical shape with, for example, an inner diameter ranging typically between about 1 nm and about 1000 nm, preferably between about 50 nm and about 500 nm.

The term "nucleic acid nanoball" as used herein is generally a concatemer containing multiple copies of a target nucleic acid molecule. These nucleic acid copies are typically arranged one after another in a continuous linear chain of nucleotides, but the nucleic acid nanoball of the present invention can also be made from any nucleic acid molecule using the methods described herein. This tandem repeat structure, along with the single-stranded nature of DNA, results in a folding configuration of the nanoball. Generally, the multiple copies of target nucleic acid molecule in the nucleic acid nanoball each contains a linker sequence with a known sequence to facilitate its amplification or sequencing. The linker sequences for each target nucleic acid molecule are usually the same, but can also be different. Nucleic acid nanoball usually includes DNA nanoball, which is also referred to as DNB (DNA nanoball) in this article.

The nucleic acid nanoball can be produced using, for example, rolling circle replication (RCA). The RCR process has been used to prepare multiple consecutive copies of M13 genome (Blanco et al., (1989) J Biol Chem 264:8935-8940). In this method, the nucleic acid is replicated in linear concatemer. Those skilled in the art can find guidance on the selection of conditions and reagents for RCR reactions in a number of references, including U.S. Patent Nos. 5,426,180, 5,854,033, 6,143,495, and 5,871,921; and for all purposes, especially for those utilizing all teachings related to the preparation of nucleic acid nanoball by RCR or other methods, these documents are incorporated herein by reference in their entirety.

The nucleic acid nanoball can be loaded on the surface of a solid support as described herein. The nanoball may be attached to the surface of the solid support by any suitable method, non-limiting examples of such method include nucleic acid hybridization, biotin-streptavidin conjugation, sulfhydryl conjugation, photoactivated conjugation, covalent conjugation, antibody-antigen, physical confinement via hydrogel or other porous polymers, etc., or combinations thereof. In some cases, the nanoball can be digested with a nuclease (e.g., DNA nuclease) to produce a smaller nanoball or fragment from the nanoball.

In some embodiments, the surface of the solid support may bear a reactive functional group that reacts with the complementary functional group on the polynucleotide molecule to form a covalent bond, e.g., by using the same techniques used to attach cDNA to microarray, which is reported in, for example, Smirnov et al. (2004), Genes, Chromosomes & Cancer, 4 0:72-77 and Beaucage (2001), Current Medicinal Chemistry, 8:1213_1244, and both of which are incorporated herein by reference. DNB can also be effectively attached to a hydrophobic surface, such as clean glass surface with low concentration of various reactive functional groups (e.g., -OH group). Attachment via covalent bonds formed between the polynucleotide molecule and reactive functional groups on the surface is also referred to herein as "chemical attachment."

In other embodiments, the polynucleotide molecule can be adsorbed to a surface. In this embodiment, the polynucleotide is immobilized through a non-specific interaction with the surface, or through a non-covalent interaction such as hydrogen bonding, van der Waals force, and the like.

In other embodiments, the nucleic acid library can be double-stranded nucleic acid fragments, which are immobilized on the surface of the solid support through a ligation reaction with an oligonucleotide immobilized on the surface of the solid support, and then a bridge amplification reaction is performed to prepare a sequencing library.

### Beneficial Effects

The present invention can achieve the following beneficial effects:
The present invention uses a saponin compound as photodamage protectant for nucleic acids, which can protect template nucleotides even at very low concentrations. The saponin compound used in the present invention has no absorption in the ultraviolet and visible light regions. Even after multiple rounds of sequencing reactions, the data quality is still very high, there is no interference with the base recognition signals, and there is no discoloration phenomenon caused by oxidization during long-term storage. By using the optimized scanning reagent of the present invention, the quality of nucleic acid sequencing, especially polynucleotide sequencing, can be greatly improved.

The embodiments of the present invention will be described in detail below with reference to the accompanying drawings and examples. However, those skilled in the art will understand that the following drawings and examples are only used to illustrate the present invention and do not limit the scope of the present invention. Various objects and advantageous aspects of the present invention will become apparent to those skilled in the art from the accompanying drawings and the following detailed description of preferred embodiments.

### Brief Description of the Drawings

Figure 1 shows the effect of the scanning reagent composition on sequencing quality in the examples.
Figure 2 shows the effect of the concentration of notoginsenoside R1 on sequencing quality in the examples.
Figure 3 shows the effect of the concentration of Trolox on sequencing quality in the examples.
Figure 4 shows the effect of the concentration of EDTA-4Na on sequencing quality in the examples.
Figure 5 shows the effect of the concentration of DTT on sequencing quality in the examples.
Figure 6 shows the effect of different concentrations of ginsenoside Rg1 on sequencing quality in the examples.

### Specific Models for Carrying Out the present invention

The embodiments of the present invention will be described in detail below with reference to examples, but those skilled in the art will understand that the following examples are only used to illustrate the present invention and should not be regarded as limiting the scope of the present invention. If the specific conditions are not specified in the examples, the conditions should be carried out according to the conventional conditions or the conditions recommended by the manufacturer. If the manufacturer of the reagents or instruments used is not indicated, they are all conventional products that can be purchased commercially.

### Example

### 1. Experimental equipment

MGISEQ-2000RS sequencer, MGIDL-200H loader, and MGISEQ-2000RS sequencing slide. The excitation wavelengths of the instruments are: 532nm and 650nm, respectively.

### 2. Reagents and raw materials used in experiments

Tris Base (powder), sodium chloride (powder), Tween-20, Tris-HCl (powder), notoginsenoside R1, Trolox, ethylenediaminetetraacetic acid tetrasodium salt dihydrate, L-dithiothreitol, ginsenoside Rg1. The above reagents were purchased from a compliant chemical reagent supply company. MGISEQ-2000RS high-throughput sequencing kit had Cat. No.1000012536 and brand of MGI. *Escherichia coli* single-stranded circular DNA was used as the template, which was the standard library reagent V3.0, and the primer sequence was: CAACTCCTTGGCTCACAGAACATGGCTACGATCCGACTT. In addition, dATP-1, which referred to adenine nucleotide with both reversible blocking group modification and Cy5 fluorescence modification; dTTP-1, which referred to thymine nucleoside with both reversible blocking group modification and ROX fluorescence modification; dGTP-1, which referred to guanine nucleotide with both reversible blocking group modification and Cy3 fluorescence modification; and dCTP-1, which referred to cytosin nucleotide with both reversible blocking group modification and EF700 fluorescence modification, were all from MGISEQ-2000RS high-throughput sequencing kit MGI.

### 3. Experimental methods:

### (a) Preparation of scanning reagent

Tris Base (powder), sodium chloride (powder), Tween-20, Tris-HCl (powder), notoginsenoside R1, Trolox, ethylenediaminetetraacetic acid tetrasodium salt dihydrate, L-dithiothreitol and ginsenoside Rg1 were dissolved in ultrapure water according to the required ratio for each set of experiments. The dissolution was performed by ultrasonic for 20 minutes, the above reagents were dissolved to form a transparent and uniform solution, which was filtered through a 0.22 micrometer filter membrane for later use.

### (b) DNA sequencing method:

Sequencing process: In the first step, DNB nanoballs were loaded onto a prepared sequencing chip.

In the second step, the prepared mixed solution of dNTP molecules was pumped into the chip, and DNA polymerase was used to add dNTPs to the complementary strand of the DNA parent strand.

In the third step, pictures were taken and scanned. Since dNTPs were modified molecules with fluorescent groups, laser with the excitation wavelength was used to take the pictures. Since laser could cause photodamage to DNA, the scanning reagent was used as a protectant in this photo-taking step. After the photos were taken, the base type was identified.

In the fourth step, the base-terminal fluorescent group and the 3'-blocking group were excised with the excision reagent and eluted, and the 3'-OH was exposed for the next round of reaction.

The MGISEQ-2000RS high-throughput sequencing kit was used, the #10 well reagent in the kit was used as the control of the scanning reagent, and PE100+70 sequencing was performed on the MGISEQ-2000RS sequencing platform according to the above experimental process. In short, in each experimental group, the scanning reagent in #10 well of the kit was replaced with the prepared scanning reagent as object of investigation, and then the Q30 decline for each cycle and the sequencing error rate curve for each cycle were calculated to evaluate the sequencing quality.

### Experiment 1: Evaluation of effect of scanning reagent composition on sequencing quality

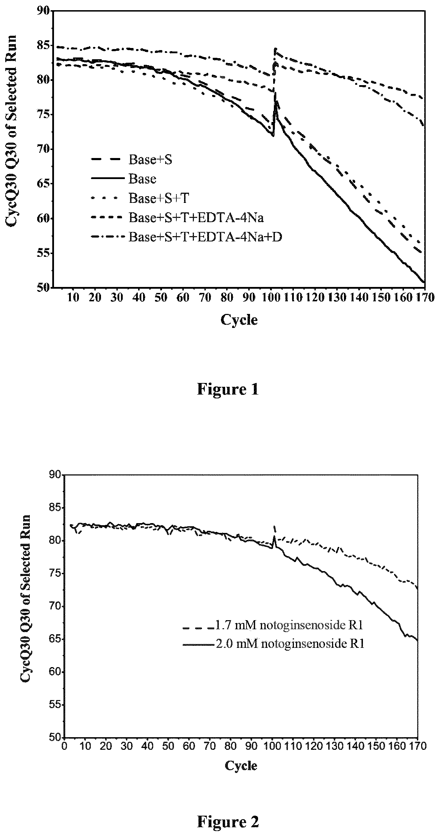

Base buffer solution was prepared according to the composition as shown in Table 1, and had a total volume of 1 L and a pH of 7.03. Scanning reagents were prepared by adding different compounds to the Base buffer, and then underwent sequencing. The composition of the scanning reagents used in each set of sequencing experiments was shown in Table 2. Among them, the concentration of notoginsenoside R1 was 1.7 mM, the concentration of Trolox was 8 mM, the concentration of EDTA-4Na was 10 mM, and the concentration of L-dithiothreitol was 8 mM. Figure 1 showed the effect of scanning reagent composition on sequencing quality.

**Table 1**

| | Compound | Amount of addition | Concentration of working solution |
|---|---|---|---|
| 1 | Tris-Base | 9.40 g | 0.077 M |
| 2 | Sodium chloride | 12.17 g | 0.200 M |
| 3 | 10% Tween | 5.00 mL | 0.05% (volume ratio) |
| 4 | Tris-HCl | 72.50 g | 0.4600 M |

**Table 2**

| Group | Composition of scanning reagent |
|---|---|
| Base+S | Base buffer solution + notoginsenoside R1 |
| Base+S+T | Base buffer solution + notoginsenoside R1 + Trolox |
| Base+S+T+EDTA-4Na | Base buffer solution + notoginsenoside R1 + Trolox + ethylenediaminetetraacetic acid tetrasodium salt dihydrate |
| Base+S+T+EDTA-4Na+D | Base buffer solution + notoginsenoside R1 + Trolox + ethylenediaminetetraacetic acid tetrasodium salt dihydrate + L-dithiothreitol |

In Figure 1, the abscissa was the number of sequencing cycle reactions, and the ordinate was the probability that the base accuracy was greater than 99.9%. The larger the ordinate value of the data in the curve, the higher the quality of the sequencing, that was, the more upward the trend, the better the data. Under the condition that the solution was Base buffer solution, the optimized results of each single factor experiment (see Experiments 2 to 6) were superimposed for testing, and the results showed that under the optimized concentration conditions, the Base+S+T+EDTA-4Na showed the best sequencing quality, and all the Q30 values for the first strand and second strand and the retention time were the best.

In Experiments 2 to 6, single-factor experiments were used to investigate the effects of the concentrations of different additives on sequencing quality, in which the composition of the Base buffer solutions used was the same as that in Example 1.

### Experiment 2: Evaluation of effect of concentration of notoginsenoside R1 on sequencing quality

Scanning reagents were obtained by adding different concentrations of notoginsenoside R1 to the Base buffer solution, and were used for sequencing. The composition of the scanning reagent used in each set of sequencing experiments were shown in Table 3.

**Table 3**

| Group | Composition of scanning reagent |
|---|---|
| 1.70 mM notoginsenoside R1 | Base buffer solution + 1.7 mM notoginsenoside R1 |
| 2.00 mM notoginsenoside R1 | Base buffer solution + 2.0 mM notoginsenoside R1 |

Figure 2 showed the effect of the concentration of notoginsenoside R1 on sequencing quality. It could be seen from Figure 2 that the sequencing quality was higher when the concentration of notoginsenoside R1 was 1.70 mM, and the second strand Q30 decreased slowly. Therefore, it was more suitable for improving sequencing quality that the concentration of notoginsenoside R1 was 1.70 mM.

### Experiment 3: Evaluation of effect of Trolox concentration on sequencing quality

Scanning reagents were obtained by adding different concentrations of Trolox to the Base buffer solution, and were used for sequencing. The composition of the scanning reagent used in each set of sequencing experiments was shown in Table 4.

**Table 4**

| Group | Composition of scanning reagent |
|---|---|
| 8 mM Trolox | Base buffer solution + 8.00 mM Trolox |
| 10 mM Trolox | Base buffer solution + 10.00 mM Trolox |

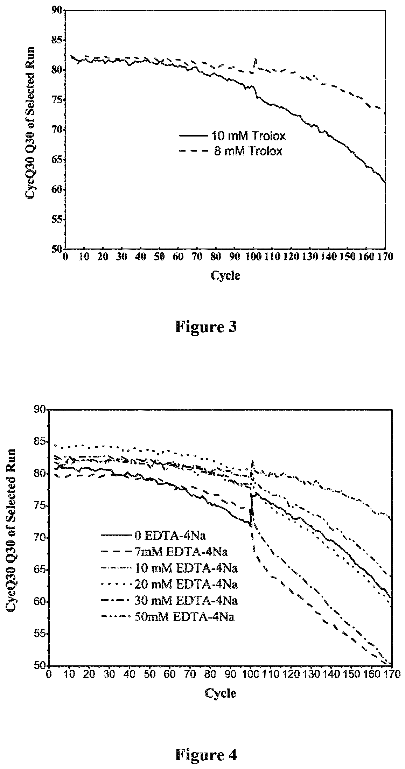

Figure 3 showed the effect of Trolox concentration on sequencing quality. It could be seen from Figure 3 that the sequencing quality is higher under the condition that the Trolox concentration was 8 mM, and the second strand Q30 decreased slowly. Therefore, it was more suitable for improving sequencing quality that the Trolox concentration was 8 mM.

### Experiment 4: Evaluation of effect of EDTA-4Na concentration on sequencing quality

Scanning reagents were obtained by adding 8 mM Trolox, 1.7 mM notoginsenoside R1 and different concentrations of EDTA-4Na to the Base buffer solution, and were used for sequencing. The concentrations of EDTA-4Na were 0 mM, 7 mM, 10 mM, 20 mM, 30 mM and 50 mM, respectively. The composition of the scanning reagent used in each set of sequencing experiments was shown in Table 5.

**Table 5**

| Group | Composition of scanning reagent |
|---|---|
| 0 EDTA-4Na | Base buffer solution + 8.00 mM Trolox + 1.70 mM notoginsenoside R1 |
| 7 mM EDTA-4Na | Base buffer solution + 8.00 mM Trolox + 1.70 mM notoginsenoside R1 + 7.00 mM EDTA-4Na |
| 10 mM EDTA-4Na | Base buffer solution + 8.00 mM Trolox + 1.70 mM notoginsenoside R1 + 10.00 mM EDTA-4Na |
| 20 mM EDTA-4Na | Base buffer solution + 8.00 mM Trolox + 1.70 mM notoginsenoside R1 + 20.00 mM EDTA-4Na |
| 30 mM EDTA-4Na | Base buffer solution + 8.00 mM Trolox + 1.70 mM notoginsenoside R1 + 30.00 mM EDTA-4Na |
| 50 mM EDTA-4Na | Base buffer solution + 8.00 mM Trolox + 1.70 mM notoginsenoside R1 + 50.00 mM EDTA-4Na |

Figure 4 showed the effect of EDTA-4Na on sequencing quality. It could be seen from Figure 4 that the sequencing quality was higher when the EDTA-4Na concentration was 10 mM, the first strand Q30 was the highest, and the second strand Q30 decreased slowly. Therefore, it was more suitable for improving sequencing quality that the concentration of EDTA-4Na was 10 mM.

### Experiment 5: Evaluation of effect of DTT concentration on sequencing quality

Scanning reagents were obtained by adding 8.00 mM Trolox, 1.70 mM notoginsenoside R1 and different concentrations of DTT to the Base buffer solution, and were used for sequencing. The concentrations of DTT were 0 mM, 5 mM, 8 mM, 10 mM and 20 mM, respectively. The composition of the scanning reagent used in each set of sequencing experiments was shown in Table 6.

**Table 6**

| Group | Composition of scanning reagent |
|---|---|
| 0 mM DTT | Base buffer solution + 8.00 mM Trolox + 1.7 mM notoginsenoside R1 |
| 5 mM DTT | Base buffer solution + 8.00 mM Trolox + 1.7 mM notoginsenoside R1 + 5.00 mM DTT |
| 8 mM DTT | Base buffer solution + 8.00 mM Trolox + 1.7 mM notoginsenoside R1 + 8.00 mM DTT |
| 10 mM DTT | Base buffer solution + 8.00 mM Trolox + 1.7 mM notoginsenoside R1 + 10.00 mM DTT |
| 20 mM DTT | Base buffer solution + 8.00 mM Trolox + 1.7 mM notoginsenoside R1 + 20.00 mM DTT |

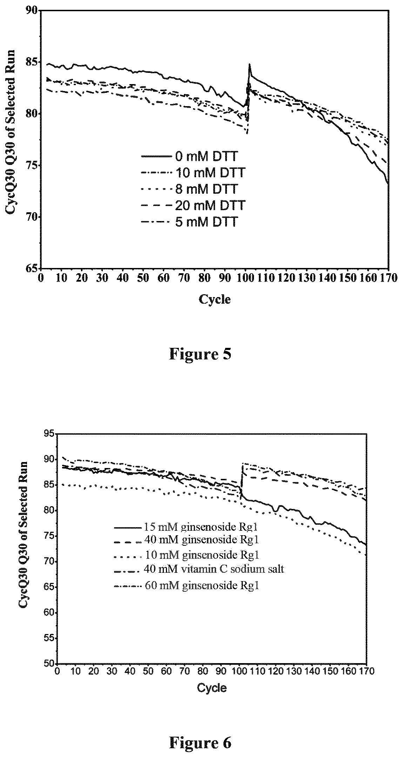

Figure 5 showed the effect of DTT concentration on sequencing quality. It could be seen from Figure 5 that the sequencing quality was higher when the concentration of DTT was 10 mM. Based on the Q30 values of the first strand and second strand, it was more suitable for improving sequencing quality that the concentration of DTT was 8 mM.

### Experiment 6: Evaluation of effect of different concentrations of ginsenoside Rg1 on sequencing quality

Based on the Base+S+T+EDTA-4Na+D solution (Base buffer solution + 1.70 mM notoginsenoside R1 + 8.00 mM Trolox + 10.00 mM ethylenediaminetetraacetic acid tetrasodium salt dihydrate + 8.00 mM L-dithiothreitol), scanning reagents were obtained by adding different concentrations of ginsenoside Rg1 to the solution and used for sequencing. The concentrations of ginsenoside Rg1 were 10 mM, 15 mM, 40 mM and 60 mM, respectively. The composition of the scanning reagent used in each set of sequencing experiments was shown in Table 7. Vitamin C sodium salt in the standard marketed kit was used as a photodamage protectant. In this experiment, the #10 well in the standard kit was used as a control.

**Table 7**

| Group | Composition of scanning reagent |
|---|---|
| 10 mM ginsenoside Rg1 | Base+S+T+EDTA-4Na+D + 10.00 mM ginsenoside Rg1 |
| 15 mM ginsenoside Rg1 | Base+S+T+EDTA-4Na+D + 15.00 mM ginsenoside Rg1 |
| 40 mM ginsenoside Rg1 | Base+S+T+EDTA-4Na+D + 40.00 mM ginsenoside Rg1 |
| 40 mM Vitamin C sodium salt | #10 well in the standard kit |
| 60 mM ginsenoside Rg1 | Base+S+T+EDTA-4Na+D + 60.00 mM ginsenoside Rg1 |

Figure 6 showed the effect of ginsenoside Rg1 concentration on sequencing quality. It could be seen from Figure 6 that under the condition that the concentration of ginsenoside Rg1 was 60.00 mM, the sequencing quality using the scanning reagent of the present invention had better effect in comparison with that of using vitamin C sodium salt as a photodamage protectant, and the Q30 values of the first stand and second strand and the retention time were all higher than those of vitamin C sodium salt in the control group.

Conclusion: The final optimized scanning reagent composition was shown in Table 8.

**Table 8**

| Compound | Amount of addition | Working concentration |
|---|---|---|
| Tris-Base | 9.40 g | 0.077 M |
| Sodium chloride | 12.17 g | 0.20 M |
| 10% Tween | 5.00 mL | 0.05% (volume ratio) |
| Tris-HCl | 72.50 g | 0.46 M |
| Trolox | 2.00 g | 8.00 mM |
| DTT | 1.23 g | 8.00 mM |
| EDTA-4Na | 4.16 g | 10.0 mM |
| Notoginsenoside R1 | 1.55 g | 1.70 mM |
| Ginsenoside Rg1 | 48.00 g | 60.00 mM |

The above examples are only used to illustrate the technical solutions of the present invention but not to limit them. Any modification or equivalent replacement of the technical solutions of the present invention without departing from the purpose and scope of the technical solutions of the present invention shall fall within the protection scope of the present invention.

## Claims

1. A scanning reagent, which comprises a saponin compound and a Tris buffer solution, the saponin compound being one or more selected from the group consisting of the following compounds:

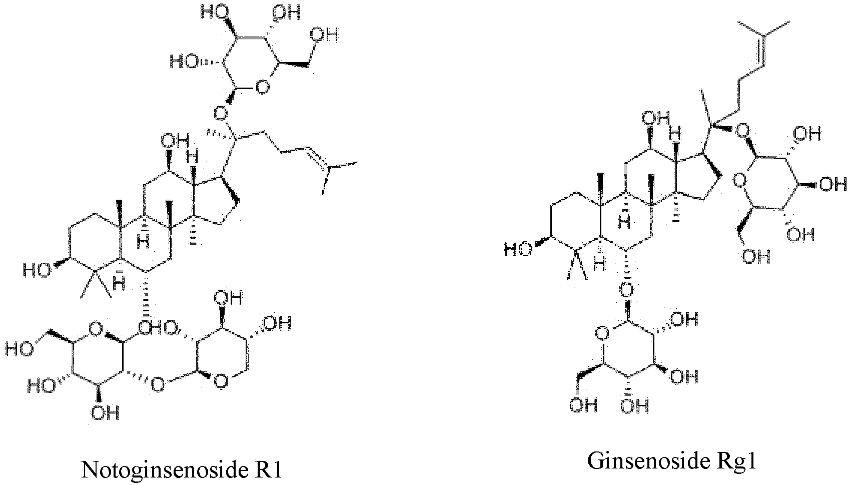

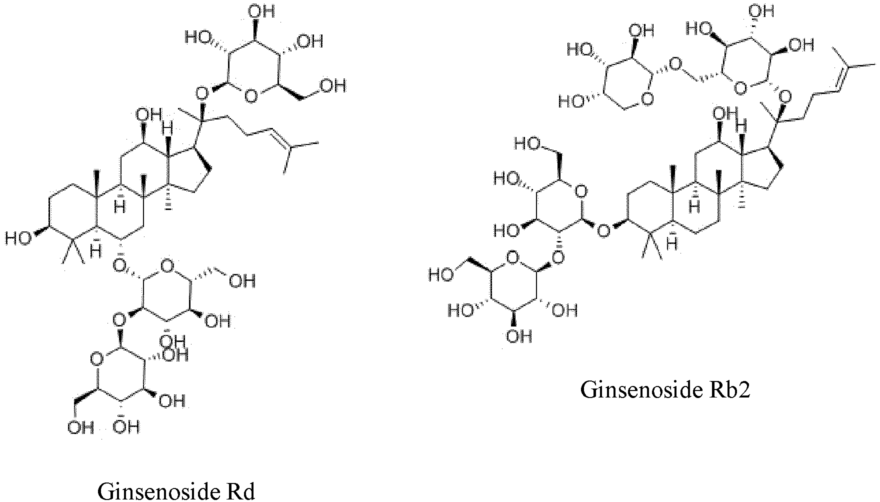

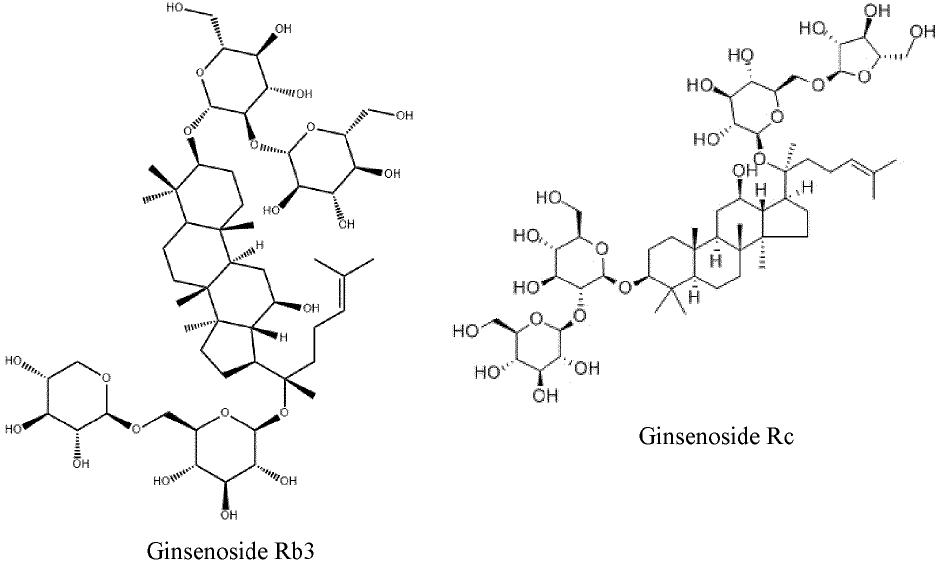

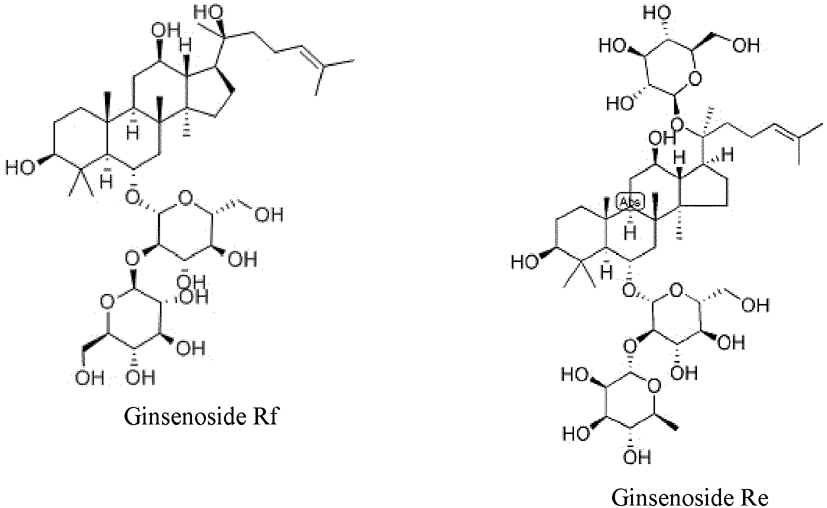

2. The scanning reagent according to claim 1, wherein the saponin compound has a concentration of 0.01-10 mM or 10-100 mM.

3. The scanning reagent according to claim 1 or 2, which further comprises an additional photodamage protectant, such as (±)-6-hydroxy-2,5,7,8-tetramethylchromane-2-carboxylic acid , ethylenediaminetetraacetic acid tetrasodium salt dihydrate, L-dithiothreitol, or any combination thereof;
preferably, the scanning reagent comprises (±)-6-hydroxy-2,5,7,8-tetramethylchromane-2-carboxylic acid;
preferably, the scanning reagent comprises (±)-6-hydroxy-2,5,7,8-tetramethylchromane-2-carboxylic acid and ethylenediaminetetraacetic acid tetrasodium salt dihydrate;
preferably, the scanning reagent comprises (±)-6-hydroxy-2,5,7,8-tetramethylchromane-2-carboxylic acid, ethylenediaminetetraacetic acid tetrasodium salt dihydrate and L-dithiothreitol;
preferably, the (±)-6-hydroxy-2,5,7,8-tetramethylchromane-2-carboxylic acid, ethylenediaminetetraacetic acid tetrasodium salt dihydrate or L-dithiothreitol in the scanning reagent each independently has a concentration of 5-50 mM.

4. The scanning reagent according to any one of claims 1 to 3, which further comprises sodium chloride and/or a DNA stabilizer (e.g., Tween-20);
preferably, the Tris buffer solution contains water, Tris, sodium chloride, Tween-20 and Tris hydrochloride.

5. A nucleic acid sequencing method, wherein the method comprises using the scanning reagent according to any one of claims 1-4.

6. Use of a saponin compound as a photodamage protectant in nucleic acid sequencing, in which the saponin compound is selected from the group consisting of notoginsenoside R1, ginsenoside Rg1, ginsenoside Rd, ginsenoside Rb2, ginsenoside Rb3, ginsenoside Rc, ginsenoside Rf, and ginsenoside Re, and their structural formulas are as shown in claim 1.

7. A kit, which comprises the scanning reagent according to any one of claims 1-4.

8. The kit according to claim 7, which further comprises: a reagent for immobilizing a nucleic acid molecule to be sequenced and a support; a primer for initiating nucleotide polymerization; and a polymerase for performing nucleotide polymerization; one or more buffer solutions; one or more washing solutions; or any combination thereof.

9. Use of the scanning reagent according to any one of claims 1 to 4 or the kit according to claim 7 or 8 for determining a sequence of a target polynucleotide.

10. A method for preparing the scanning reagent according to any one of claims 1 to 4, comprising dissolving each component of the scanning reagent in ultrapure water to prepare a transparent and uniform solution, and then filtering the solution.
